(19) Europäisches Patentamt

European Patent Office

Office européen des brevets

(11) **EP 1 627 628 A1**

(12) **DEMANDE DE BREVET EUROPEEN**

(43) Date de publication:
**22.02.2006 Bulletin 2006/08**

(51) Int Cl.:
***A61K 8/87*** *(2006.01)*   ***A61K 8/04*** *(2006.01)*
***A61Q 5/06*** *(2006.01)*

(21) Numéro de dépôt: **05291359.7**

(22) Date de dépôt: **24.06.2005**

(84) Etats contractants désignés:
**AT BE BG CH CY CZ DE DK EE ES FI FR GB GR
HU IE IS IT LI LT LU MC NL PL PT RO SE SI SK TR**
Etats d'extension désignés:
**AL BA HR LV MK YU**

(30) Priorité: **01.07.2004 FR 0451397**

(71) Demandeur: **L'OREAL**
**75008 Paris (FR)**

(72) Inventeurs:
• **Samain, Henri**
**91570 Bièvres (FR)**

• **Rollat-Corvol, Isabelle**
**75017 Paris (FR)**
• **Gawtrey, Jonathan**
**92100 Boulogne (FR)**
• **Schultze, Xavier**
**77340 Pontault-Combault (FR)**

(74) Mandataire: **Bourdeau, Françoise**
**L'OREAL - D.I.P.I.**
**25-29 Quai Aulagnier**
**92600 Asnières (FR)**

(54) **Composition capillaire pressurisée comprenant au moins un polymère filmogène élastomère**

(57)    L'invention a pour objet une composition capillaire pressurisée comprenant au moins un polymère filmogène élastomère et au moins un agent propulseur, ainsi que l'utilisation de ces compositions pour le coiffage des cheveux.

EP 1 627 628 A1

**Description**

L'invention a pour objet une composition capillaire pressurisée comprenant au moins un polymère filmogène élastomère et au moins un agent propulseur, ainsi que l'utilisation de ces compositions pour le coiffage des cheveux.

Les compositions de coiffage, telles que les laques et les sprays, conditionnées sous forme de spray aérosol sont généralement composées d'une phase liquide comprenant, dans un milieu cosmétiquement acceptable alcoolique ou hydroalcoolique, au moins un polymère filmogène, et d'un agent propulseur qui est un gaz liquéfié sous pression réduite ou dissous dans la phase liquide.

Dans le domaine des produits capillaires, on cherche à fabriquer des laques en aérosol ne comportant aucun composé organique volatil tel que l'éthanol ou le diméthyléther, et ce pour des raisons essentiellement écologiques, tout en conservant de bonnes propriétés de mise en forme et de maintien de la coiffure.

Le problème posé par l'invention est d'obtenir de bonnes conditions de pulvérisation ainsi qu'une laque présentant des propriétés de mise en forme et de maintien tout à fait équivalentes à celles des laques de l'état de la technique et ne comportant en outre aucun composé organique volatil.

La demanderesse a constaté que l'utilisation, dans des compositions capillaires pressurisées, de certains polymères filmogènes élastomères, conduit à des formulations facilement pulvérisables, en particulier lorsqu'elles contiennent une forte proportion d'eau. Ces compositions s'appliquent aisément sur les cheveux et, de façon surprenante, conduisent beaucoup plus rapidement à l'aspect d'un chevelure sèche que les polymères de l'art antérieur testés. Les polymères filmogènes élastomères présentent un pouvoir coiffant satisfaisant et s'éliminent facilement par un simple shampooing. Le brossage des cheveux traités ne conduit pas à un effet de poudrage mais laisse les cheveux doux et brillants.

L'invention a pour objet une composition capillaire, conditionnée dans un dispositif aérosol, comprenant, dans un milieu cosmétiquement acceptable, au moins un polymère élastomère filmogène filmogène non ionique, anionique, amphotère ou cationique avec des groupements fluorés choisi de telle sorte que le matériau obtenu par séchage de ce polymère, à température ambiante et à un taux d'humidité relative de 55%, présente un profil mécanique défini par au moins :

a) un taux d'allongement à la rupture (e) supérieur ou égal à 800% ;
b) une recouvrance instantanée (Ri) supérieur ou égal à 75% après un allongement de 150% ;
c) une recouvrance (R300s) à 300 secondes supérieur à 80%, et

au moins un agent propulseur.

Elle a en outre pour objet l'utilisation d'une telle composition capillaire pour le coiffage des cheveux.

DESCRIPTION DETAILLEE DES MODES DE REALISATION DE L'INVENTION

Au sens de la présente invention, on entend par film obtenu par séchage à température ambiante (22°C $\pm$ 2°C) et à un taux d'humidité relative de 55 % $\pm$ 5%, le film obtenu dans ces conditions à partir d'un mélange à 6 % de matière active (m.a.) de polymère filmogène élastomérique dans un mélange 30 % en poids d'éthanol et de 70 % en poids d'eau, par rapport au poids total alcool+eau, la quantité de mélange étant adaptée pour obtenir dans une matrice en téflon, un film d'épaisseur de 500 $\mu$m $\pm$ 50 $\mu$m. Le séchage est poursuivi jusqu'à ce que le poids du film n'évolue plus, ce qui représente environ 12 jours. Les polymères filmogène solubles ou partiellement solubles dans l'éthanol sont testés dans l'éthanol seul. Les autres polymères sont testés dans l'eau seule, sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22°C $\pm$ 2 °C et un taux d'humidité relative de 50 % $\pm$ 5 %.

Les éprouvettes sont étirées à la vitesse de 20mm/min et la distance entre les mors est de 50 $\pm$ 1 mm.

Pour déterminer la recouvrance instantanée ($R_i$), on procède comme suit :

- on étire l'éprouvette de 150 % ($e_{max}$) c'est-à-dire 1,5 fois sa longueur initiale ($l_0$),
- on relâche la contrainte en imposant une vitesse de retour égale à la vitesse de traction, soit 20mm/min, et on mesure l'allongement de l'éprouvette en pourcentage, après retour à charge nulle ($e_i$).

La recouvrance instantanée en % ($R_i$) est donnée par la formule ci-après :

$$R_i = ((e_{max} - e_i)/ e_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($e_{300s}$).

La recouvrance à 300 secondes en % ($R_{300s}$) est donnée par la formule ci-après :

$$R_{300s} = ((e_{max} - e_{300s})/ e_{max}) \times 100$$

De façon avantageuse, le ou les polymères de la composition selon l'invention, éventuellement associé(s) à un agent plastifiant et/ou un agent de filmification, sont tels qu'ils forment, dans les conditions des tests ci-dessus, un film d'allongement à la rupture allant de 800 % à 3000 % ; de recouvrance instantanée de 75 % à 100 % ; et une recouvrance à 300secondes allant de 85 % à 100 %.

Dans les compositions conformes à l'invention, le polymère filmogène élastomérique ou le mélange de polymères filmogènes élastomériques est, de préférence, présent à une concentration allant de 0,05 % à 20 % en poids, plus préférentiellement de 0,1 % à 15 % en poids, et par exemple de 0,25 % à 10 % en poids par rapport au poids total de la composition.

De manière avantageuse, le polymère filmogène élastomérique est choisi dans le groupe comprenant les polyuréthanes, les alcools polyvinyliques, les polymères comprenant au moins un motif (méth)acrylique, leurs associations. Il peut se présenter sous forme d'homopolymère ou de copolymère. En particulier, il se présente sous forme non réticulée dans la composition.

Si nécessaire, la composition peut, en outre comprendre un agent plastifiant et/ou un agent facilitant la filmification du ou des polymères élastomériques sur les matières kératiniques, dont la fonction est de modifier les propriétés du ou des polymères élastomériques. Un tel agent auxiliaire de filmification peut être choisi parmi tous les composés connus de l'homme du métier comme étant susceptibles de remplir la fonction recherchée, et être notamment choisi parmi les agents plastifiants et les agents de coalescence. Le ou les polymères filmogènes élastomériques, éventuellement associés à un agent plastifiant et/ou un agent facilitant la filmification sont aptes à former un film, après évaporation du milieu cosmétique. Cette évaporation peut être faite à l'air libre ou en apportant de la chaleur, par exemple à l'aide d'un séchoir.

Comme exemple d'agent plastifiant et/ou facilitant la filmification sur les matières kératiniques, on peut utiliser ceux décrits dans le document FR-A-2 782 917. En particulier, cet agent est choisi parmi les plastifiants ou agents de coalescence usuels, tels que:

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, le pentylène glycol,
- les esters de glycérol,
- les dérivés de propylène glycol et en particulier le propylène glycol phényléther, le propylène glycol diacétate, le dipropylène glycol butyléther, le tripropylène glycol butyléthér, le propylène glycol méthyléther, le dipropylène glycol éthyléther, le tripropylène glycol méthyléther et le diéthylène glycol méthyléther, le propylène glycol butyléther,
- les esters d'acides notamment carboxyliques, tels que des citrates, des phtalates, des adipates, des carbonates, des tartrates, des phosphates, des sébaçates,
- leurs mélanges.

La quantité d'agent plastifiant et/ou d'agent de filmification peut être choisie par l'homme du métier sur base de ses connaissances générales, de manière à obtenir un système polymérique (polymères élastomériques + agent plastifiant et/ou agent de filmification) conduisant à un film ayant les propriétés mécaniques souhaitées, tout en conservant à la composition les propriétés cosmétiques recherchées. En pratique, cette quantité varie de 0,01 % à 25 % du poids total de la composition et mieux de 0,01 % à 15 %.

Avantageusement, dans les compositions selon l'invention, le polymère filmogène est de préférence soluble dans un milieu hydroalcoolique.

On entend au sens de la présente invention par "composés solubles" dans un milieu donné, des composés (monomères ou polymères) qui, introduits dans ledit milieu à 25°C, à une concentration en poids égale à 10 %, si besoin est, neutralisés, permettent l'obtention d'une solution macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.

On entend par "composés dispersibles" dans un milieu, des composés (monomères ou polymères) qui, introduits dans ledit milieu à 25°C à une concentration en poids supérieure ou égale à 1 % en poids permettent l'obtention d'une dispersion homogène non transparente.

Avantageusement, le matériau obtenu par séchage du polymère, à température ambiante et à un taux d'humidité relative de 55%, est de préférence hydrosoluble et / ou hydrodispersible.

Avantageusement, une solution hydro alcoolique de 5 % en poids de polymère filmogène, à 30 % en poids d'eau complétée à 100 % en poids par de l'alcool, présente une viscosité inférieure à 200 cps à 25°C et supérieure à 20 cps.

La viscosité est mesurée au viscosimètre Rhéomat 180.

Dans les compositions conformes à l'invention, le polymère filmogène élastomère ou le mélange de polymères filmogènes élastomères est, de préférence, présent à une concentration allant de 0,05 % à 20 % en poids, plus préférentiellement de 0, 1 % à 15 % en poids, et par exemple de 0,25 % à 10 % en poids par rapport au poids total de la composition.

De manière avantageuse, le polymère filmogène élastomère est choisi dans le groupe comprenant les polyuréthannes, les alcools polyvinyliques, les polymères comprenant au moins un motif (méth)acrylique, leurs mélanges. Il peut se présenter sous forme d'homopolymère ou de copolymère. En particulier, il se présente sous forme non réticulée dans la composition.

Les polymères filmogènes élastomères utiles dans la composition de l'invention sont connus de la technique. Ils peuvent par exemple être synthétisés selon la méthode décrite dans la demande de brevet FR 2 815-350.

DISPOSITIF AEROSOL

On entend par milieu cosmétiquement acceptable selon l'invention un milieu constitué d'eau ou d'un ou plusieurs solvants organiques cosmétiquement acceptables, tels que par exemple les alcools inférieurs en C 1 - C4 en particulier l'éthanol, l'isopropanol, le tertio-butanol et le n-butanol, et les alkylèneglycols comme le propylèneglycol, ou encore un mélange d'eau et d'un ou de plusieurs solvants organiques cosmétiquement acceptables.

La composition du milieu cosmétiquement acceptable et sa proportion dans la composition capillaire finale sont de préférence, telles que la teneur globale de la composition capillaire en composés organiques volatils (VOC, volatil organic compounds) est au plus égale à 55 % en poids.

L'agent propulseur peut être n'importe quel gaz liquéfiable utilisé habituellement dans des dispositifs aérosol, tels que le diméthyléther, les alcanes en C3-5, le 1 , 1 - difluoroéthane, les mélanges de diméthyléther et d'alcanes en C3-5, et les mélanges de 1 , 1 -difluoroéthane et de diméthyléther et/ou d'alcanes en C3-5.

On préfère tout particulièrement utiliser comme agent propulseur le diméthyléther et les alcanes en C3-C5, et en particulier le propane, le n-butane et l'isobutane.

Le rapport pondéral phase liquide/agent propulseur des compositions capillaires pressurisées de la présente invention est de préférence compris entre 50 et 0,05, et en particulier entre 25 et 1,5.

Les compositions capillaires de la présente invention peuvent contenir en outre un ou plusieurs ingrédients cosmétiques ou de formulation, utilisés habituellement dans le domaine cosmétique.

On peut citer à titre d'exemples de tels ingrédients les polymères filmogènes anioniques, cationiques, non-ioniques ou amphotères, différents des copolymères diblocs amphiphiles décrits ci-dessus, les silicones volatiles ou non volatiles, les agents tensioactifs anionique, cationiques, amphotères ou non-ioniques, les agents épaississants, les agents nacrants, les filtres UV, les agents anti-radicalaires, les parfums, les agents conservateurs, les pigments et colorants, les agents d'ajustement du pH, les agents solubilisants, les agents plastifiants, les agents anti-mousse, les cires et huiles, les vitamines, des agents conditionneurs et les particules organiques ou minérales, synthétiques ou d'origine naturelle.

Si le dispositif aérosol servant à conditionner les compositions de l'invention est à deux compartiments, il peur être constitué par un bidon aérosol externe comportant une poche interne soudée hermétiquement à une valve. La composition est introduite dans la poche interne et un gaz comprimé est introduit entre la poche et le bidon à une pression suffisante pour faire sortir le produit sous forme d'un spray à travers l'orifice d'une buse. Un tel dispositif est commercialisé sous le nom EP SPRAY par la société EP-SPRAY SYSTEM SA. Ledit gaz comprimé est utilisé de préférence sous une pression comprise entre 1 et 12 bars, mieux encore comprise entre 9 et 11 bars.

Bien entendu l'homme du métier veillera à choisir ce ou ces éventuels additifs complémentaires et/ou leurs quantités de manière à ce que les propriétés avantageuses intrinsèques des compositions de l'invention ne soient pas altérées par la ou les adjonctions envisagées.

Les compositions capillaires sont de préférence des laques pour cheveux ou des mousses.

L'invention est illustrée à l'aide des exemples suivants.

Illustration N° 1 de l'invention : Laque mono compartimenté propulsée par un gaz liquéfié dans un dispositif aérosol classique :

| Polyuréthane (NMDEA[1] / PTMO 2900[2] / IPDI[3]) Fomblin diol 1000[4] - 2 / 0.5 / 3 / 0.5) | 3g% MA |
|---|---|
| Ethanol | 10g |
| Eau | 52g |
| DME | 35g |
| [1] - N-méthyldiéthanolamine<br>[2] - Poly (tétraméthylène oxyde) ayant une masse moyenne en poids de 2900<br>[3] - Isophoronediisocanate<br>[4] - Perfluoroéther | |

L'utilisation de cette laque permet d'obtenir un très bon niveau de fixation des cheveux ainsi qu'une excellente tenue de la coiffure dans le temps.

Illustration N° 2 de l'invention : Laque bi compartimenté propulsée à l'air comprimé :

| Polyuréthane (NMDEA[1] / PTMO 2900[2] / IPDI[3] Fomblin diol 1000[4] - 2 0.5/3/0.5) | 3g% MA |
|---|---|
| Eau | 97g |

Cet exemple de formulation est conditionné dans un dispositif aérosol à poche, commercialisé sous le nom de EP spray ce dispositif de distribution aérosol comprend un ensemble constitué d'une poche soudée hermétiquement à une valve et d'un diffuseur à buse tourbillonnaire. La valve est fixée sur un bidon aérosol classique.

La poche est remplie avec l'exemple de formulation et de l'air comprimé est introduit entre la poche et le bidon à une pression suffisante pour faire sortir le produit sous forme d'un spray.

Pression du gaz comprimé : entre 1 à 12 Bars, de préférence 9 à 11 Bars.


**Revendications**

1.  Composition capillaire, conditionnée dans un dispositif aérosol, comprenant, dans un milieu cosmétiquement acceptable, au moins un polymère élastomère filmogène filmogène non ionique, anionique, amphotère ou cationique avec des groupements fluorés choisi de telle sorte que le matériau obtenu par séchage de ce polymère, à température ambiante et à un taux d'humidité relative de 55%, présente un profil mécanique défini par au moins :

    a) un taux d'allongement à la rupture (e) supérieur ou égal à 800% ;
    b) une recouvrance instantanée ($R_i$) supérieur ou égal à 75% après un allongement de 150% ;
    c) une recouvrance ($R_{300s}$) à 300 secondes supérieur à 80%, et

    au moins un agent propulseur.

2.  Composition selon la revendication 1, **caractérisée en ce que** le polymère filmogène élastomérique est soluble dans un milieu aqueux ou hydroalcoolique.

3.  Composition selon la revendication 1 ou 2, **caractérisée en ce qu'**entre 30 % et 80 % d'humidité relative, le taux d'allongement à la rupture du film obtenu soit compris entre 400 et 1200% et /ou sa recouvrance instantanée soit comprise entre 57 et 93%.

4.  Composition selon l'une quelconque des revendications précédentes, **caractérisée par** le fait le polymère filmogène est soluble dans un milieu hydro alcoolique.

5.  Composition selon l'une quelconque des revendications précédentes, **caractérisée par le fait que** le matériau obtenu par séchage du polymère, à température ambiante et à un taux d'humidité relative de 55% est hydrosoluble et / ou hydrodispersible.

**6.** Composition selon la revendication 2, **caractérisée en ce qu'**une solution hydro alcoolique de 5 % en poids de polymère filmogène, à 30 % en poids d'eau complétée à 100 % en poids par de l'alcool, présente une viscosité inférieure à 200 cps à 25°C et supérieure à 20 cps.

**7.** Composition selon l'une quelconque des revendications précédentes, **caractérisée en ce que** le polymère filmogènes élastomère ou le mélange de polymères filmogènes élastomères est présent à une concentration allant de 0,05 % à 20 % en poids, plus préférentiellement de 0,1 % à 15 % en poids, par rapport au poids total de la composition.

**8.** Composition selon l'une quelconque des revendications précédentes, **caractérisée en ce que** le polymère filmogène est choisi dans le groupe comprenant les polyuréthannes, les alcools polyvinyliques, les polymères comprenant au moins un motif (méth)acrylique ou leurs mélanges.

**9.** Composition capillaire selon l'une quelconque des revendications précédentes, **caractérisée par le fait que** l'agent propulseur est choisi parmi le diméthyléther, les alcanes en C3-5, le 1,1-difluoroéthane, les mélanges de diméthyléther et d'alcanes en C3-5, et les mélanges de 1,1-difluoroéthane et de diméthyléther et/ou d'alcanes en C3-5.

**10.** Composition capillaire selon l'une quelconque des revendications précédentes, **caractérisée par le fait qu'**elle contient en outre des additifs cosmétiques et/ou des adjuvants de formulation tels que les polymères filmogènes anioniques, cationiques, non-ioniques ou amphotères, solubles ou dispersibles dans un milieu aqueux ou hydroalcoolique, les silicones volatiles ou non volatiles, les agents tensioactifs anionique, cationiques, amphotères ou non-ioniques, les agents épaississants, les agents nacrants, les filtres UV, les agents anti-radicalaires, les parfums, les agents conservateurs, les pigments et colorants, les agents d'ajustement du pH, les agents solubilisants, les agents plastifiants, les agents anti-mousse, les cires et huiles, les vitamines, des agents conditionneurs et les particules organiques ou minérales, synthétiques ou d'origine naturelle.

**11.** Composition capillaire selon l'une quelconque des revendications précédentes, **caractérisée par le fait qu'**elle contient au plus 55 % en poids de composés organiques volatils.

**12.** Utilisation d'une composition capillaire selon l'une quelconque des revendications précédentes, pour le coiffage des cheveux.

**Office européen**
**des brevets**

## RAPPORT DE RECHERCHE EUROPEENNE

Numéro de la demande

EP 05 29 1359

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 815 350 A (OREAL) 19 avril 2002 (2002-04-19) * le document en entier * ----- | 1-10 | A61K7/11 |
| X | FR 2 785 182 A (OREAL) 5 mai 2000 (2000-05-05) * page 9, ligne 20 - page 25, ligne 30; revendication 1 * ----- | 1-10 | |
| X | FR 2 801 204 A (OREAL) 25 mai 2001 (2001-05-25) * revendications 1,11,12 * ----- | 1-10 | |
| X | FR 2 786 392 A (OREAL) 2 juin 2000 (2000-06-02) * page 12, ligne 17-23; revendication 1 * ----- | 1-10 | |
| X | FR 2 786 391 A (OREAL) 2 juin 2000 (2000-06-02) * page 22, ligne 10-17; revendication 1 * ----- | 1-10 | |
| X | FR 2 801 200 A (OREAL) 25 mai 2001 (2001-05-25) * page 6, ligne 9-15; revendication 1 * ----- | 1-10 | DOMAINES TECHNIQUES RECHERCHES (Int.Cl.7) A61K |
| X | FR 2 801 203 A (OREAL) 25 mai 2001 (2001-05-25) * page 11, ligne 20-27; revendication 1 * ----- | 1-10 | |
| X | FR 2 815 854 A (OREAL) 3 mai 2002 (2002-05-03) * page 9, ligne 1-7; revendication 1 * ----- | 1-10 | |
| X | FR 2 848 430 A (OREAL) 18 juin 2004 (2004-06-18) * page 34, ligne 5-15; revendications 1,20,21 * ----- | 1-10 | |

Le présent rapport a été établi pour toutes les revendications

| Lieu de la recherche | Date d'achèvement de la recherche | Examinateur |
|---|---|---|
| Munich | 24 octobre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

**ANNEXE AU RAPPORT DE RECHERCHE EUROPEENNE
RELATIF A LA DEMANDE DE BREVET EUROPEEN NO.**

EP 05 29 1359

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-10-2005

| Document brevet cité au rapport de recherche | | Date de publication | Membre(s) de la famille de brevet(s) | | Date de publication |
|---|---|---|---|---|---|
| FR 2815350 | A | 19-04-2002 | AU | 9569201 A | 29-04-2002 |
| | | | EP | 1326908 A1 | 16-07-2003 |
| | | | WO | 0232978 A1 | 25-04-2002 |
| | | | JP | 2004511637 T | 15-04-2004 |
| | | | US | 2004052753 A1 | 18-03-2004 |
| FR 2785182 | A | 05-05-2000 | AU | 746529 B2 | 02-05-2002 |
| | | | AU | 6345599 A | 22-05-2000 |
| | | | CA | 2316398 A1 | 11-05-2000 |
| | | | CN | 1292675 A | 25-04-2001 |
| | | | EP | 1043969 A1 | 18-10-2000 |
| | | | WO | 0025736 A1 | 11-05-2000 |
| | | | JP | 3646149 B2 | 11-05-2005 |
| | | | JP | 2002528479 T | 03-09-2002 |
| | | | PL | 341481 A1 | 23-04-2001 |
| | | | RU | 2203101 C2 | 27-04-2003 |
| FR 2801204 | A | 25-05-2001 | AU | 768024 B2 | 27-11-2003 |
| | | | AU | 1711201 A | 30-05-2001 |
| | | | BR | 0008909 A | 04-12-2001 |
| | | | CA | 2360430 A1 | 25-05-2001 |
| | | | CN | 1336815 A | 20-02-2002 |
| | | | CZ | 20012513 A3 | 13-02-2002 |
| | | | EP | 1146847 A1 | 24-10-2001 |
| | | | WO | 0135911 A1 | 25-05-2001 |
| | | | JP | 2003513999 T | 15-04-2003 |
| | | | MX | PA01007255 A | 09-04-2002 |
| | | | PL | 348862 A1 | 17-06-2002 |
| | | | RU | 2219900 C2 | 27-12-2003 |
| | | | US | 6749837 B1 | 15-06-2004 |
| FR 2786392 | A | 02-06-2000 | AU | 746140 B2 | 18-04-2002 |
| | | | AU | 1276600 A | 13-06-2000 |
| | | | BR | 9907724 A | 17-10-2000 |
| | | | CN | 1295458 A | 16-05-2001 |
| | | | EP | 1051145 A1 | 15-11-2000 |
| | | | WO | 0030593 A1 | 02-06-2000 |
| | | | JP | 2002530309 T | 17-09-2002 |
| | | | PL | 341932 A1 | 07-05-2001 |
| | | | RU | 2200534 C2 | 20-03-2003 |
| | | | US | 6497864 B1 | 24-12-2002 |
| FR 2786391 | A | 02-06-2000 | AU | 745846 B2 | 11-04-2002 |
| | | | AU | 1276700 A | 13-06-2000 |
| | | | BR | 9907723 A | 17-10-2000 |
| | | | CN | 1295459 A | 16-05-2001 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 05 29 1359

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-10-2005

| Document brevet cité au rapport de recherche | | Date de publication | Membre(s) de la famille de brevet(s) | | Date de publication |
|---|---|---|---|---|---|
| FR 2786391 | A | | EP | 1051146 A1 | 15-11-2000 |
| | | | WO | 0030594 A1 | 02-06-2000 |
| | | | JP | 2002530310 T | 17-09-2002 |
| | | | JP | 2004307516 A | 04-11-2004 |
| | | | PL | 341980 A1 | 07-05-2001 |
| | | | RU | 2183449 C2 | 20-06-2002 |
| | | | US | 6391292 B1 | 21-05-2002 |
| FR 2801200 | A | 25-05-2001 | AU | 1710601 A | 30-05-2001 |
| | | | BR | 0015691 A | 09-07-2002 |
| | | | EP | 1233739 A1 | 28-08-2002 |
| | | | WO | 0135910 A1 | 25-05-2001 |
| | | | JP | 2003533433 T | 11-11-2003 |
| | | | MX | PA02004994 A | 18-09-2002 |
| FR 2801203 | A | 25-05-2001 | AU | 1711301 A | 30-05-2001 |
| | | | BR | 0015762 A | 06-08-2002 |
| | | | CA | 2391640 A1 | 25-05-2001 |
| | | | CN | 1391462 A | 15-01-2003 |
| | | | EP | 1233741 A1 | 28-08-2002 |
| | | | WO | 0135915 A1 | 25-05-2001 |
| | | | JP | 2003527339 T | 16-09-2003 |
| | | | MX | PA02004996 A | 18-09-2002 |
| | | | PL | 356209 A1 | 14-06-2004 |
| FR 2815854 | A | 03-05-2002 | AU | 758105 B2 | 13-03-2003 |
| | | | AU | 7828901 A | 02-05-2002 |
| | | | BR | 0105690 A | 25-06-2002 |
| | | | CA | 2360899 A1 | 30-04-2002 |
| | | | CN | 1350837 A | 29-05-2002 |
| | | | CZ | 20013637 A3 | 12-06-2002 |
| | | | EP | 1201224 A1 | 02-05-2002 |
| | | | HU | 0104562 A2 | 28-11-2002 |
| | | | JP | 2002145727 A | 22-05-2002 |
| | | | MX | PA01010998 A | 12-08-2004 |
| | | | PL | 350444 A1 | 06-05-2002 |
| | | | US | 2002086040 A1 | 04-07-2002 |
| | | | ZA | 200108318 A | 02-05-2002 |
| FR 2848430 | A | 18-06-2004 | BR | 0305902 A | 17-05-2005 |
| | | | CN | 1506035 A | 23-06-2004 |
| | | | EP | 1444974 A2 | 11-08-2004 |
| | | | JP | 2004196801 A | 15-07-2004 |

EPO FORM P0460

Pour tout renseignement concernant cette annexe : voir Journal Officiel de l'Office européen des brevets, No.12/82